# EUROPEAN PATENT APPLICATION

(11) **EP 4 799 610 A1**
(43) Date of publication of application: **02.09.2026**
(21) Application number: 25160978.0
(22) Date of filing: 28.02.2025
(51) Int. Cl.: A61K 9/08, A61K 31/519, A61K 47/10, A61K 47/12

(54) **TOPICAL PHARMACEUTICAL COMPOSITION COMPRISING SILDENAFIL**

(71) Applicant: Dr. August Wolff GmbH & Co. KG Arzneimittel, 33611 Bielefeld (DE)
(72) Inventor: Giller, Sven, 33611 Bielefeld (DE); Dr. Häuser, Silvia, 33611 Bielefeld (DE); Dr. Lauterbach, Andreas, 33611 Bielefeld (DE)
(74) Representative: Weickmann & Weickmann PartmbB

(57) **Abstract**

The present invention relates to a pharmaceutical composition comprising sildenafil or a pharmaceutically acceptable salt thereof, at least one alcohol, and an aqueous buffer, and said pharmaceutical composition for use in a method of preventing and/or treating hair loss and/or female sexual dysfunction and/or erectile dysfunction.

## Description

The present invention relates to a pharmaceutical composition comprising sildenafil or a pharmaceutically acceptable salt thereof, at least one alcohol, and an aqueous buffer, and said pharmaceutical composition for use in a method of preventing and/or treating hair loss and/or female sexual dysfunction and/or erectile dysfunction.

A local increase of blood flow and microcirculation in the skin or regions just below the skin can promote the treatment of various disorders such as hair loss, female sexual dysfunction, and erectile dysfunction.

The causes of hair loss are manifold and range from age-related hair loss to genetic or hormonal disorders, autoimmune disorders, metabolic disorders, including malnutrition, to infections, traumas, tumors and medications such as cytostatic and anticoagulants. Hair loss comprises different disease states such as androgenetic alopecia, telogen effluvium, alopecia areata, frontal fibrosing alopecia, and lichen planopilaris.

Hair loss caused by hormones such as androgens is called androgenetic alopecia (alopecia androgenetica or AGA) and is considered to be the most common cause of hair loss, especially in men but also in women. A distinction can be made between male and female hair loss patterns. Other common forms of non-scarring alopecia comprise telogen effluvium (TE), a diffuse hair loss, and the acutely occurring inflammatory condition of circular hair loss, also known as alopecia areata (AA).

Lichen planopilaris is an inflammatory autoimmune disease of the skin causing destruction of hair follicles and scarring alopecia. Frontal fibrosing alopecia is a clinical variant of lichen planopilaris affecting mainly women after menopause.

Stimulating blood flow in the scalp promotes the local microcirculation and may improve nutrient delivery and oxygenation of hair follicles. Different studies have by now demonstrated that local blood flow stimulation may promote hair growth and be effective in the prevention and/or treatment of hair loss (Choi et al., Biochem Biophys Res. Commun. (2018) 505, 685-691, and Mostafa et al., Anthropology (2023) 11, 310).

Female sexual dysfunction comprises female sexual arousal disorder with different disorder types such as genital arousal disorder, subjective arousal disorder, and combined arousal disorder. Similarly to the treatment of erectile dysfunction, local or systemic increase of blood flow has been used as therapeutic approach to treating female sexual dysfunction.

Sildenafil is an active pharmaceutical ingredient (API) and phosphodiesterase type 5 (PDE5) inhibitor which is approved for treatment of erectile dysfunction and pulmonary arterial hypertension. Sildenafil promotes vasodilation and subsequently an increased local blood circulation.

Sildenafil acts on the NO/cGMP signaling pathway to promote vasodilation. Endothelial NOS (eNOS) produces NO, which acts explicitly as an endogenous vasodilator, diffuses into the vascular cells and mediates smooth muscle relaxation by binding to guanylyl cyclase. Guanylyl cyclase converts GTP to cGMP, which activates cGMP-dependent protein kinases. These protein kinases then phosphorylate various ion channels in the endoplasmic reticulum (ER), thereby sequestering calcium and preventing calcium mobilization within the cell. As a result, the smooth muscle cells relax (Jennifer Witek; Anand D. Lakhkar., Treasure Island (FL): StatPearls Publishing; 2024 Jan). The NO/cGMP signaling cascade is dampened by the action of cGMP-specific phosphodiesterase type 5 (PDE5), which breaks down cGMP by converting it to GMP. Sildenafil is a specific PDE5 inhibitor and thereby promotes cGMP signaling and vasodilation. The present invention is not limited to this mechanism.

Sildenafil can be formulated as a salt such as sildenafil citrate or sildenafil lactate.

Sildenafil citrate is available as oral tablet, oral suspension, and intravenous solution (e.g. Viagra^{®}, Revatio^{®}).

Unfortunately, sildenafil features a broad systemic adverse effect profile due to its systemic availability from oral application or fast permeable formulations. Systemic adverse effects include anaemia, fluid retention, insomnia, anxiety, headache, migraine, tremor, paraesthesia, burning sensation, hypoaesthesia, retinal haemorrhage, visual impairment, vertigo, flushing, epistaxis, diarrhoea, and dyspepsia.

Topical application generally enables the administration of an API to the skin for local treatment and reduction or avoidance of systemic availability and adverse effects.

For local treatment of the skin, the API needs to exhibit penetration into the skin. Additionally, for treatment of hair follicle associated diseases the API may also need to exhibit penetration into the hair follicles.

To obtain local penetration of active pharmaceutical ingredients (APIs) into the skin and the hair follicle the API needs to be dissolved in the formulation. Optionally, organic solvents such as alcohols may promote the penetration route via the hair follicle due to sebum solubilization (Grice et al., J. Pharm. Sci. (2010) 99(2), 712-718).

Sildenafil is soluble in organic solvents but exhibits poor solubility in water. Further, solubility of sildenafil in water has been reported to be pH-dependent: while it is already only poorly soluble at pH 3-4, the solubility has been reported to decrease steeply above a pH of 4 (Wang et al., Int. J. Pharm. (2008) 352, 217-224).

In addition, aqueous solutions of lower concentrations of sildenafil citrate have been reported to show an increased photodegradation (Eichhorn et al., Journal of Mass Spectometry (2012) 47, 701-711) that is not appropriate for topical application to the skin due to sun light exposure of the skin.

Consequently, most reported liquid compositions of sildenafil employ relatively high amounts of organic solvents.

Choi et al. (Biochem Biophys Res. Commun. (2018) 505, 685-691) report the use of 0.1% sildenafil and 1% sildenafil in 30% ethanol and 70% polyethylene glycol as a vehicle for hair growth in mice. Mostafa et al. (Anthropology (2023) 11, 310) describe the application of 1% sildenafil citrate in 100% ethanol to treat hair loss in women.

EP 2081547 B1 describes solutions of sildenafil for transmucous application wherein the solution contains 45% ethanol. EP 3119201 B1 claims a solution of sildenafil containing at least 20% ethanol as well as less than 20% acetone. US 10211534 B2 claims a pharmaceutical composition of at least 35% ethanol and dissolved sildenafil citrate.

Thus, reported topical formulations of sildenafil commonly comprise high amounts of organic solvents such as ethanol.

On the other hand, permeation across the skin may promote systemic availability of the API. Permeation is based on transdermal diffusion whereby the API may pass the skin barrier and reach the systemic blood circulation, leading to systemic availability and consequently systemic adverse effects. Generally, high amounts of organic solvents such as alcohols and solubilizers may enhance the permeation rate of APIs due to their interaction with the skin.

Thus, a need remains for stable and photostable topical formulations of sildenafil or pharmaceutically acceptable salts thereof, wherein sildenafil is stably dissolved to allow its local penetration into the skin and hair follicles, but which do not promote strong permeation of sildenafil across the skin.

Further, lower amounts of alcoholic components, a broad acidic pH value range of 3.0-6.0, and a higher amount of water are beneficial regarding local skin compatibility.

This task is surprisingly solved by the present invention, which provides solution formulations of sildenafil or pharmaceutically acceptable salts thereof meeting the requirements above, with lower amounts of alcoholic components, reduced transdermal diffusion rates and improved photostability compared to formulations reported in the prior art.

Thus, the present invention relates to a pharmaceutical composition comprising
(A) 0.001-2.5% (w/w) of an active agent selected from sildenafil or a pharmaceutically acceptable salt thereof,
(B) 5-25% (w/v) of at least one alcohol, and
(C) at least 75% (w/v) of an aqueous buffer.

As used herein, if not stated otherwise, amounts given in % (w/w) or % (w/v) are always indicated with respect to the total weight or volume of the respective composition, such as the pharmaceutical composition of the invention.

The active agent (A) is preferably any pharmaceutically acceptable salt of sildenafil, particularly sildenafil citrate or sildenafil lactate. In a preferred embodiment, the active agent (A) is sildenafil citrate.

It is preferred that the active agent (A) is stably dissolved in the pharmaceutical composition. As used herein, stably dissolved means that the active agent (A) is completely dissolved and molecularly dispersed in the composition at 15-40°C and does not precipitate when kept at 15-40°C for an extended period of time, i.e., multiple days to weeks. The absence of precipitation of the active agent (A) may be assessed by methods known in the art, such as by assessing the macroscopic and/or microscopic appearance of the composition, which may include visual inspection for solid precipitate or crystals and/or assessing or measuring the turbidity of the composition.

The active agent (A) is present in the pharmaceutical composition of the invention in an amount of 0.001-2.5% (w/w). Preferably, the concentration of active agent (A) is 0.01-2.5% (w/w), more preferably 0.1-2.5% (w/w), more preferably 0.5-2.5% (w/w), even more preferably 0.5-2.25% (w/w), even more preferably 0.75-2.00% (w/w), even more preferably 0.75-1.50% (w/w) and particularly 0.75-1.25% (w/w), with respect to the total weight of the pharmaceutical composition.

The at least one alcohol (B) comprised in the pharmaceutical composition of the invention may be a single alcohol or a mixture of two or more alcohols.

Preferably, the at least one alcohol (B) is selected from C₂-C₄-alcohols having 1-3 hydroxy groups, or any mixtures thereof, more preferably selected from C₂-C₄-alcohols having 1-2 hydroxy groups, or any mixtures thereof. It is particularly preferred that the at least one alcohol (B) is a mixture or a single alcohol, preferably a single alcohol, selected from C₂-C₃-alcohols having 1 hydroxy group. It is further preferred that the one or more alcohols are saturated alcohols and unbranched in the case of C₄-alcohols. Preferably, the hydroxy groups are the only functional groups present in the alcohols.

It is particularly preferred that the at least one alcohol (B) is selected from the group consisting of ethanol, isopropyl alcohol, 1-propanol, glycerol, propylene glycol, and 1,3-propanediol, or a mixture thereof. In a more preferred embodiment, the at least one alcohol (B) is isopropyl alcohol, ethanol, or a mixture thereof, in particular isopropyl alcohol.

In preferred embodiments, the concentration of the at least one alcohol (B) is 10-25% (w/v), more preferably 10-20% (w/v), with respect to the total volume of the pharmaceutical composition.

All simple alcohols in the composition are preferably taken into account to determine the proportion of component (B). The total concentration of simple alcohols in the pharmaceutical composition of the invention is therefore preferably 5-25% (w/v), more preferably 10-25% (w/v), even more preferably 10-20% (w/v), with respect to the total volume of the pharmaceutical composition. As used herein, simple alcohols are hydrocarbons bearing only hydroxy groups as functional groups.

Preferably, in the pharmaceutical composition of the invention, the weight ratio of active agent (A) to the at least one alcohol (B) is 1:4-1:50, preferably 1:6-1:35, more preferably 1:8-1:25.

The aqueous buffer (C) comprised in the pharmaceutical composition of the present invention preferably has a pH of 3.0-6.0, more preferably 3.1-4.9, even more preferably 3.3-4.5.

The main solvent present in the aqueous buffer (C) is water. In a preferred embodiment, the aqueous buffer comprises water as the only solvent.

Preferably, the aqueous buffer (C) is a buffer solution comprising at least one weak acid and at least one weak base as buffering agents. Preferably, the weak base is the conjugate base of the weak acid. Weak acids and their pKₐ values and weak bases and their pK_{b} and conjugate pKₐ values are known in the art. The weak acid and/or weak base may be monoprotic or polyprotic, preferably monoprotic, and are preferably pharmaceutically acceptable compounds. The weak acid and/or weak base are preferably organic compounds, more preferably carboxylic acids and carboxylates, respectively. The buffer solution may comprise a single weak acid and its conjugate base, one weak acid and a non-conjugate weak base, or mixtures of multiple weak acids and weak bases.

The conjugate base of a weak acid may be comprised in the aqueous buffer (C) in the form of any pharmaceutically acceptable salt with sufficient solubility at 20°C to be completely dissolved in the aqueous buffer (C) at the desired concentration. Preferably, the salt has a solubility in water at 20°C of ≥ 0.01 g/ml, more preferably of ≥ 0.1 g/ml, particularly of ≥ 1 g/ml. Methods for preparing an aqueous buffer are known in the art and may comprise dissolving the weak acid and/or the weak base or a salt thereof in water at the desired concentration and adjusting the pH of the solution using a strong acid or base, such as HCl or NaOH. If the buffer comprises a weak acid and its conjugate base, equivalent buffer solutions may be prepared by dissolving only the weak acid, only the conjugate base of the weak acid or a salt thereof, or both the weak acid and its conjugate base or a salt thereof in water and adjusting the pH of the solution to the desired pH, which is preferably in the buffering range of the weak acid/base pair.

It is preferred that the aqueous buffer (C) has a buffering range which comprises the pH of the aqueous buffer (C). The buffering range of a buffer solution may be calculated based on the pKₐ of the at least one weak acid and optionally the pKₐ of the conjugate acid of the at least one weak base by methods known in the art. For example, if a single weak acid/base pair is present in the buffer, the buffering range may be estimated as pKₐ ± 1, with a preferred range of pKₐ ± 0.5. It is thus preferred that the buffering range of (C) overlaps with the pH range of 3.0-6.0, more preferably 3.1-4.9, even more preferably 3.3-4.5. It is particularly preferred that at least one pKₐ of the least one weak acid and optionally of the conjugate acid of the at least one weak base is in the pH range of 3.0-6.0, more preferably 3.1-4.9, even more preferably 3.3-4.5.

Accordingly, it is preferred that the at least one weak acid and optionally at least one weak base is chosen such that at least one pKₐ of the weak acid and optionally of the conjugate acid of the at least one weak base is close to the desired pH of the aqueous buffer (C), pH(C), preferably that the pKₐ is within pH(C) ± 1, more preferably within pH(C) ± 0.5.

Preferably, the at least one weak acid of the aqueous buffer solution is selected from lactic acid, citric acid, succinic acid, acetic acid, tartaric acid, etc. or any mixtures thereof.

It is further preferred that the at least one weak base is selected from pharmaceutically acceptable salts of lactate, citrate, succinate, acetate, tartrate, etc. or any mixtures thereof, particularly that the at least one weak base is the conjugate base of the at least one weak acid or a pharmaceutically acceptable salt thereof.

Preferably, the aqueous buffer (C) is a lactate buffer. A lactate buffer is a buffer solution wherein the main buffering agent is the conjugate pair of lactic acid and lactate. The lactate buffer may be a pure lactate buffer comprising no further buffering agents or may be a mixed buffer comprising further buffering agents apart from lactic acid and lactate. If the lactate buffer is a mixed buffer, it is preferred that its total lactate concentration is higher than the concentration of any other buffering agent, preferably higher than the combined concentration of all other buffering agents present in the composition. Preferably, the aqueous buffer (C) comprises lactic acid and a lactate salt, wherein the lactate salt is particularly a pharmaceutically acceptable lactate salt. The lactate salt preferably comprises a cation selected from Na, Ca, Mg and Zn. More preferably, the lactate salt is sodium lactate.

It is preferred that the aqueous buffer (C) comprises the buffering agents, i.e., the one or more weak acids and their conjugate bases, in a total concentration of 0.001-2 M, preferably 0.01-1 M, more preferably 0.01-1.0 M, particularly 0.1-0.8 M. Here, total concentration refers to the combined total molar concentration (M = mol/l) of all present species of all buffering agents, such as the one or more weak acids and the one or more weak bases, e.g. base anions, in the buffer solution.

In a preferred embodiment, the aqueous buffer (C) is a lactate buffer having a total lactate concentration of 0.001-2 M, preferably 0.01-1 M, more preferably 0.01-1.0 M, particularly 0.1-0.8 M. As used herein, total lactate concentration is the sum of the concentrations of all lactate species present in the buffer, including particularly deprotonated and protonated lactate (= lactic acid).

In a preferred embodiment, the aqueous buffer (C) is a lactate buffer comprising 0.1 - 4.1% (w/v) lactic acid and 0.05 - 3.1% (w/v) sodium lactate, preferably 0.5 - 3.0% (w/v) lactic acid and 0.2 - 2.5% (w/v) sodium lactate, in particular 1.0 - 2.5% (w/v) lactic acid and 0.5 - 2.0% (w/v) sodium lactate.

In a particularly preferred embodiment, the aqueous buffer (C) is a lactate buffer having a total lactate concentration of 0.001-2 M, preferably 0.01-1 M, more preferably 0.01-1.0 M, particularly 0.1-0.8 M and a pH of 3.0-6.0, more preferably 3.1-4.9, even more preferably 3.3-4.5.

The pharmaceutical composition of the present invention is preferably liquid at room temperature, such as at 20°C. Preferably, the pharmaceutical composition has a viscosity of 0.01-2000 mPa·s at 20°C. The viscosity may be measured according to DIN 53019-1:2008-09 using a rheometer, such as an MCR 301 rheometer (Anton Paar), at 20°C with a shear rate of 20 s⁻¹ using a cone-plate geometry.

It is further preferred that the pharmaceutical composition of the invention has a pH of 3.0-6.0, more preferably 3.1-4.9, even more preferably 3.3-4.5. Preferably, the pH of the pharmaceutical composition is set by the aqueous buffer and remains stable after storage at a temperature of 25°C-40°C for at least 6 months.

Preparation of the pharmaceutical composition of the invention may comprise dissolving the weak base and weak acid of the buffer system in water to provide the aqueous buffer (C), mixing the aqueous buffer (C) with the at least one alcohol (B) at their final weight ratio and dissolving sildenafil citrate in the resulting solution while stirring and/or agitating the liquid. Surprisingly, it was found that sildenafil citrate can be dissolved immediately in a solution of (B) and (C) as described above, despite the low amount of organic solvent and even at a pH value in the range of 3.0-6.0, preferably 3.1-4.9.

Thus, the inventors have found that a solution comprising components (B) and (C) of the pharmaceutical composition of the present invention surprisingly dissolves high amounts of the active agent (A), particularly sildenafil citrate. Surprisingly, sildenafil citrate can be dissolved in a pharmaceutical composition of the invention in a concentration of up to 2.5% (w/w), while using only 25% (w/v), 20% (w/v) or even less organic solvent such as isopropyl alcohol and ethanol.

Additionally, the solubility of sildenafil citrate in a composition according to the invention is surprisingly nearly constant over a pH range of pH 3.1 - 4.9. This finding is in contrast to the strong decrease of solubility of sildenafil citrate in water a pH range of pH 4 - 6 as reported previously.

Also, it was found that the pharmaceutical composition of the invention is and remains stable during storage at 25°C and 40°C for at least 6 months. In this context, stable means that the concentration of sildenafil citrate in the pharmaceutical composition, which may be measured e.g. by HPLC, remains in the range of 95 - 105% with respect to its starting value, the pH remains essentially constant, e.g. within a range of ± 0.1, preferably ±0.05. Further impurities remain at negligible levels, preferably below 0.1 g/100 g, more preferably below 0.05 g/100 g.

Further, as shown in the Examples, the inventors surprisingly found that the pharmaceutical composition of the invention shows an improved stability under light exposure compared to an aqueous solution of sildenafil citrate, which shows significant degradation.

The pharmaceutical composition of the invention is preferably for topical application, more preferably for topical application to the skin. Topical application is the local administration to a body part, preferably of liquid and/or semi-solid dosage forms that contain one API or several APIs. The pharmaceutical composition may be applied to various areas of the skin of human or animal subjects, preferably of human subjects. In a preferred embodiment, the pharmaceutical composition is for topical application to the scalp. In an alternative embodiment, the pharmaceutical composition is for topical application to the intimate area, such as to the female or male genital tract.

The pharmaceutical composition of the invention may be present in any dosage form suitable for the intended use. Preferably, the pharmaceutical composition is present in a liquid or semi-solid dosage form, more preferably in a liquid dosage form. Preferred liquid dosage forms include solutions, lotions, emulsions, suspensions, foams, or shampoos. Preferred semi-solid dosage forms are gels, creams, ointments, pastes, or poultices.

Solutions are liquid preparations in which the active ingredients and excipients are dissolved in a solvent, which comprises an aqueous buffer and an alcohol according to the present invention.

Lotions are preparations for external application to the skin with a liquid to semi-solid consistency.

Emulsions are mixtures of normally immiscible liquids and typically comprise aqueous and oily phases according to the invention. Emulsions may be stabilized by emulsifiers known in the art.

Suspensions are heterogeneous mixtures of substances consisting of a solid and a liquid phase. The solid phase can settle over time at the bottom of the storage vessel. Therefore, suspensions should often be shaken immediately before use.

Foam formulations are typically provided in specialized dosing systems that generate the foam upon application, e.g. pressurized aerosol foams. The formulations typically comprise surfactants to enable creation of stable foams.

Shampoos are typically liquid or semi-liquid preparations provided for hair care.

Gels consist of gelled liquids and are produced with suitable gelling agents such as cellulose derivatives, starches, carbomers, gelatine, xanthan, bentonite, agar, tragacanth, carrageen, alginate and/or pectin.

Creams are semi-solid preparations that are usually intended for application to the skin or mucous membrane. Creams are multiphase preparations consisting of a lipophilic and an aqueous phase.

Ointments are semi-solid preparations for external application. They consist of a single-phase base in which solid or liquid substances may be dispersed. This is in contrast to creams, which are multiphase and consist of a lipophilic and an aqueous phase.

Pastes are semi-solid preparations which typically comprise finely powdered solid substances.

A poultice is a soft moist mass which is typically spread on a cloth for external application to the skin.

In preferred embodiments, the pharmaceutical composition according to the invention comprises at least one further active agent. The at least one further active agent may be selected depending on the indication to be treated. In other embodiments, the further active agent is not included in the composition according to the invention, but is administered in a corresponding combination. When the composition according to the invention is administered in combination with another composition comprising a further active agent, the administration of the two compositions is preferably carried out independently of each other in accordance with the respective dosage instructions of the two compositions. Simultaneous administration of the two compositions is also possible.

In preferred embodiments, the pharmaceutical composition of the invention comprises at least one further active agent in an amount of 0.001-10.0% (w/w), preferably 0.005-7.50% (w/w), more preferably 0.01-5.0% (w/w), particularly 0.1-3.0% (w/w), with respect to the total weight of the composition.

Preferably, the at least one further active agent is selected from the group comprising NO-donors, menthol, biotin, zinc PCA, caffeine, niacinamide, panthenol, ectoine, ubiquinone-10, taurine, echinacea, tocopheryl acetate and combinations thereof.

Nitric oxide (NO) donors may act synergistically with sildenafil, as NO increases cGMP production by activation of guanylyl cyclase while sildenafil decreases cGMP breakdown by inhibiting PDE5. Among other effects, the resulting increased cGMP levels cause smooth muscle relaxation and vasodilation through downstream effectors, thus increasing local blood flow. While systemic administration of NO-donors in combination with PDE5 inhibitors may lead to dangerous adverse effects including a drop in blood pressure, such effects may be avoided through topical use on a limited area of the body, particularly if transdermal permeation is limited. Preferably, NO-donors may be selected from L-arginine (the physiological precursor of NO), nitroglycerin, isosorbide dinitrate, sodium nitroprusside, and Minoxidil.

Menthol is a monocyclic monoterpene alcohol and can be added to the composition according to the invention as the active agent that stimulates blood flow. In addition, menthol can have a refreshing sensory stimulating effect on the skin.

Biotin, also known as vitamin B7 or vitamin H, is a water-soluble vitamin in the B complex. Biotin can strengthen the skin.

Zinc PCA is the zinc salt of L-pyrrolidone carboxylate and can be added to the composition according to the invention as a substance with an antimicrobial effect.

Caffeine is a methylxanthine alkaloid belonging to the class of methylxanthines. It is a bitter crystalline substance, can be considered a purine derivative and is chemically related to the adenine and guanine bases of deoxyribonucleic acids and ribonucleic acid. The IUPAC name of caffeine is 1,3,7-trimethyl-3,7-dihydro-1H-purine-2,6-dione. Caffeine is known for a variety of pharmacological effects. In particular, it is well known for its stimulating effect on the central nervous system. There is a growing body of evidence for a positive influence on a range of different dysfunctions.

Niacinamide (also known as nicotinamide) is the amide of nicotinic acid and is also referred to as vitamin B3. Niacinamide leads to a reduction in oxidative stress.

Panthenol is a provitamin that is converted into pantothenic acid (vitamin B5) in the body. The latter is part of coenzyme A and is therefore important for skin metabolism. When panthenol is applied, skin elasticity and moisture are further improved. It also relieves itching and inflammation and promotes wound healing.

Ectoine is a cyclic amino acid that exists in zwitterionic form in aqueous solutions. It has been shown to protect against UV radiation and may be helpful in the treatment of inflammatory diseases.

Ubiquinone-10 (Q10 or coenzyme Q10) is a quinone derivative. Q10, which belongs to the ubiquinone pool, is considered an antioxidant and has a stabilizing effect on the skin.

Taurine or 2-aminoethanesulfonic acid also has a further stabilizing effect on the skin as an antioxidant.

Echinacea has a soothing effect on the skin and relieves itching and tension. It can also stimulate blood circulation in the skin.

Tocopheryl acetate has antioxidant properties and, according to the invention, has a further stabilizing effect on the skin.

In preferred embodiments, the pharmaceutical composition of the invention further comprises at least one additive selected from the group consisting of refatteners, preservatives, stabilizers, antioxidants, rheology modifiers, thickeners, coloring agents and any combination thereof. Suitable additives are for example mentioned in Ph. Eur. 04/2022:0927 Liquid preparations for cutaneous application.

Refatteners, also known as refattening agents or superfattening agents, are lipophilic substances that can prevent an adverse effect on the epidermal barrier function. Examples of refatteners are lanolin, squalene, liquid paraffin, vegetable oils, silicones and cetyl palmitate.

Preservatives are substances used for preservation by killing microorganisms that decompose the composition and/or by inhibiting their growth. Preferably, preservatives can be selected from the group consisting of benzoic acid, benzoic acid derivatives, sorbic acid, sorbic acid derivatives, salicylic acid, salicylic acid derivatives, phenoxyethanol, parabens and combinations thereof. In preferred embodiments, sodium benzoate and/or potassium sorbate may be used as a preservative in the pharmaceutical composition according to the invention. Sodium benzoate releases benzoic acid in a slightly acidic environment and potassium sorbate releases sorbic acid. Both acids are said to have an antimicrobial effect.

Stabilizers can protect light-sensitive components from radiation, with UV absorbers such as benzophenone derivatives being preferred.

An antioxidant or antioxidant agent is a chemical compound that slows down or completely prevents oxidation of other components in the pharmaceutical composition according to the invention. For example, citric acid, ascorbic acid and butylated hydroxyanisole can be used as antioxidants.

Rheology modifiers and thickeners can help to improve the application properties of the composition according to the invention. The addition of common salt (sodium chloride) can be considered as a rheology modifier and thickening agent. By adding common salt, the flowability of the composition according to the invention can be influenced within certain limits and adjusted to the necessary degree. In addition, cellulose derivatives or polyacrylates can be used as thickeners.

Colorants are optionally used to impart a characteristic color to the pharmaceutical composition according to the invention for improved distinction from other compositions and to improve patient acceptance and/or compliance.

In a further aspect, the present invention provides a pharmaceutical product comprising the pharmaceutical composition of the invention as defined herein. The pharmaceutical product is preferably for topical application, particularly to the skin. Preferred dosage forms for the pharmaceutical product are liquid and/or semi-solid dosage forms as defined above, particularly semi-solid dosage forms.

Preferably, the pharmaceutical product of the invention comprises the pharmaceutical composition of the invention in an amount of at least 20 % (w/w), preferably at least 30% (w/w), more preferably at least 50% (w/w).

In preferred embodiments, the pharmaceutical product further comprises at least one solvent, pharmaceutically acceptable excipient and/or additive in addition to the pharmaceutical composition of the invention. Preferred additives are defined above, and suitable excipients and solvents are known in the art. The at least one solvent may particularly comprise lipophilic solvents such as oils. It is preferred that the at least one solvent does not comprise a simple alcohol as defined above.

In a preferred embodiment, the pharmaceutical product is an emulsion and/or cream comprising the pharmaceutical composition of the invention as aqueous phase and at least one oil as lipophilic phase, wherein the emulsion and/or cream is preferably stabilized by at least one emulsifier.

In an alternative embodiment, the pharmaceutical product is a gel comprising the pharmaceutical composition of the invention and at least one gelling agent and/or thickener as defined above.

As mentioned above, the pharmaceutical composition of the invention is preferably for topical application. Topical administration facilitates achievement of local effects, such as local treatment of the skin and hair follicles or local increase of blood flow. Topical administration may feature a reduced systemic adverse effects profile if permeation and consequently systemic availability are avoided.

An important parameter determining whether a topically administered composition comprising an API has predominantly local effects or if the API becomes available systemically is the transdermal diffusion rate. To achieve targeted local effects and avoid systemic adverse effects, a low transdermal diffusion rate is desired.

As a model system for measuring transdermal diffusion rates, Strat-M^{®} membranes are wellestablished in the field (Haq et al. Int J Pharm (2018) 547(1-2) 432-437). Franz diffusion cells may be used to assess diffusion of an active agent across the membranes, typically under infinite dosing conditions.

The inventors have surprisingly found through experiments that pharmaceutical compositions according to the invention show distinctly lower transdermal diffusion rates of sildenafil citrate across Strat-M^{®} membranes than known vehicles of sildenafil citrate consisting of either 100% ethanol or 70% polyethylene glycol and 30% ethanol. It was further found that pharmaceutical compositions of the invention also exhibit lower transdermal diffusion rates of sildenafil citrate across Strat-M^{®} membranes than formulations of sildenafil citrate with (unbuffered) water and alcohols like isopropyl alcohol and ethanol.

In particular, the transdermal diffusion of sildenafil citrate, always measured for 1% sildenafil citrate solutions applied to Strat-M^{®} membranes under infinite dosing for 24 h, was found to be around 38000 pg/cm² for 100% ethanol as solvent and around 3000 pg/cm² for EtOH:PEG 30:70, around 420 pg/cm² for solvents comprising water and isopropyl alcohol or ethanol and around 90-190 pg/cm² for compositions according to the present invention.

Thus, in preferred embodiments, the pharmaceutical composition of the invention has a transdermal diffusion rate of active agent (A) of less than 300 pg/cm², preferably less than 250 pg/cm², more preferably less than 200 pg/cm², in 24 hours across a Strat-M membrane under infinite dosing.

Thus, the pharmaceutical compositions of the invention advantageously achieve good solubility of sildenafil citrate at pH values suitable for topical administration to the skin combined with high storage and light stability and a favorable transdermal diffusion behavior which reduces systemic availability and may increase local effects. As a result, the pharmaceutical compositions of the invention surprisingly provide improved local delivery of sildenafil or a pharmaceutically acceptable salt thereof while reducing the risk of systemic adverse effects.

In a further aspect, the present invention provides a pharmaceutical composition as described herein above for use in a method of preventing and/or treating hair loss and/or female sexual dysfunction and/or erectile dysfunction.

In a particularly preferred embodiment, the pharmaceutical composition is for use in a method of preventing and/or treating hair loss, preferably wherein the hair loss is selected from androgenetic alopecia, telogen effluvium, alopecia areata, frontal fibrosing alopecia, and lichen planopilaris. The method of preventing and/or treating hair loss may comprise topically applying the pharmaceutical composition to the scalp.

In an alternative embodiment, the pharmaceutical composition is for use in a method of preventing and/or treating female sexual dysfunction, preferably wherein the female sexual dysfunction comprises female sexual arousal disorder, preferably selected from genital arousal disorder, subjective arousal disorder, and/or combined arousal disorder. The method of preventing and/or treating female sexual dysfunction may comprise topically applying the pharmaceutical composition to the female genital tract, particularly to vaginal tissue.

The dosage of the pharmaceutical composition used according to the invention depends on the indication to be treated and the patient to be treated. Among other parameters, age, weight, height, gender, other medications taken, especially on a regular basis, pregnancy and/or preexisting conditions may play a role here. Based on such parameters, the dosage can be determined by a specialist. The compositions can be administered for the treatment and/or prevention of acute and/or chronic conditions.

The present invention shall be further illustrated in more detail but not limited by the following figures and examples.

### Brief description of the drawings

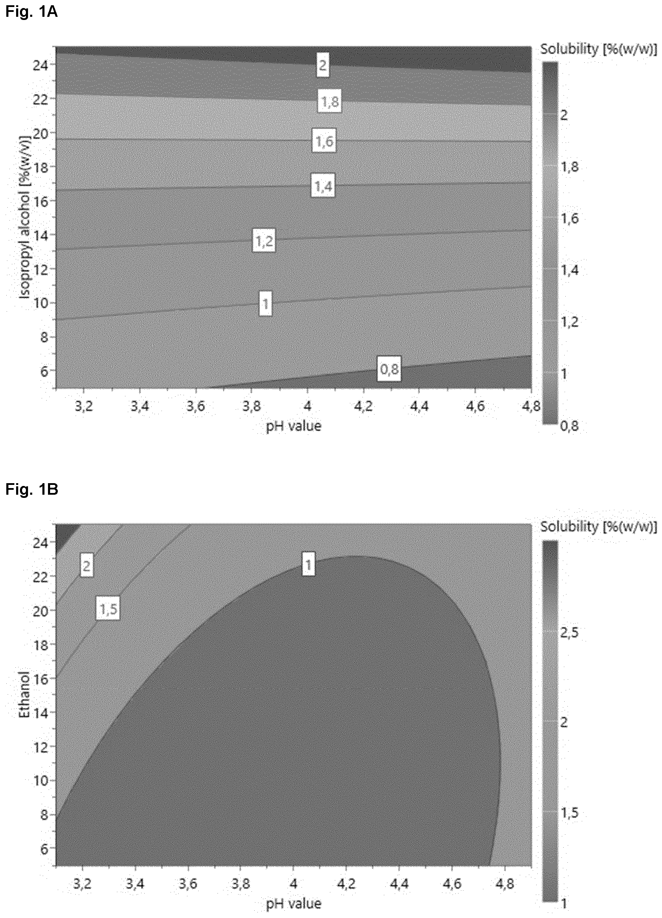

- **Fig. 1:**: Solubility of sildenafil citrate [% (w/w)] in combinations of 5 - 25% (w/v) alcohol with a lactate buffer in a pH range of 3.1 - 4.8. (A) Solubility of sildenafil citrate in solutions of 5-25% (w/v) isopropyl alcohol and lactate buffer at pH 3.1 - 4.8. (B) Solubility of sildenafil citrate in solutions of 5 - 25% (w/v) ethanol and lactate buffer at pH 3.1 - 4.9.

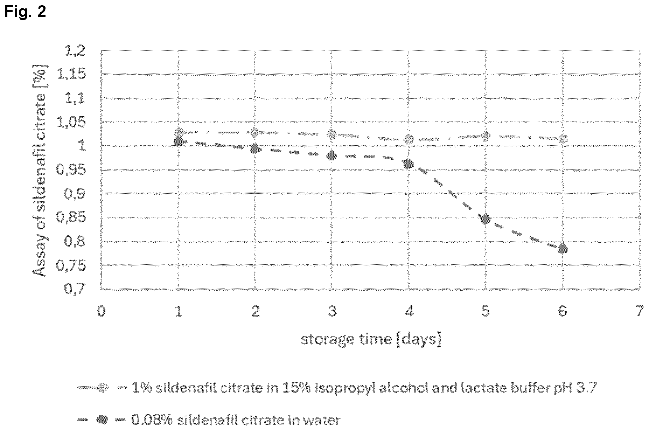

- **Fig. 2:**: Assay results of 1% (w/v) sildenafil citrate dissolved in 15% (w/v) isopropyl alcohol and lactate buffer at pH 3.7 compared to 0.08% (w/v) sildenafil citrate dissolved in water under light stress conditions over six days.

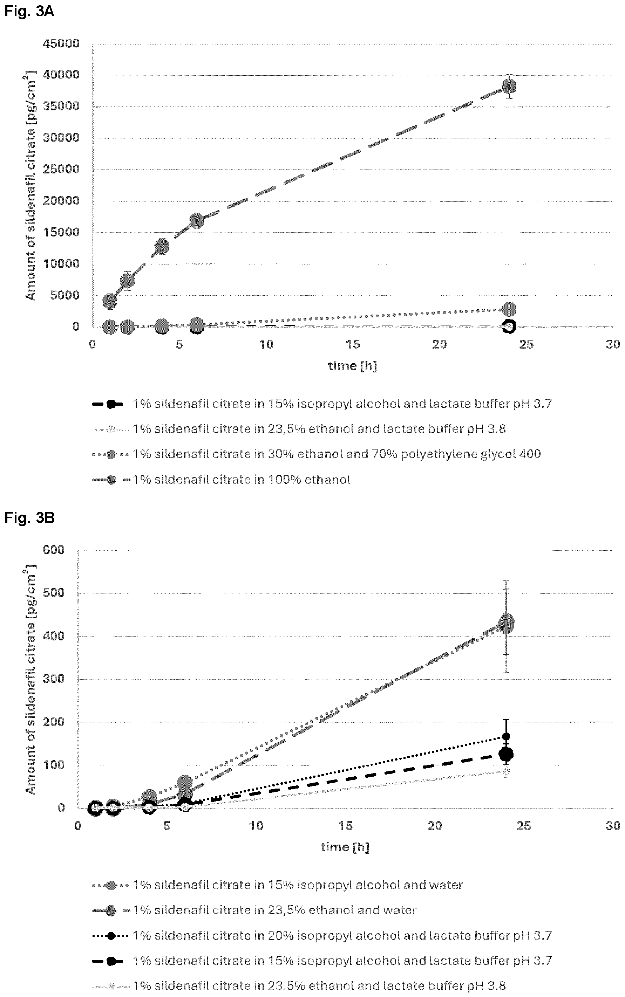

- **Fig. 3:**: Transdermal diffusion rates of different sildenafil citrate formulations across Strat-M^{®} membranes. (A) Transdermal diffusion rates of 1% (w/v) sildenafil citrate in 15% (w/v) isopropyl alcohol and lactate buffer at pH 3.7, 1% (w/v) sildenafil citrate in 23.5% (w/v) ethanol and lactate buffer at pH 3.8, 1% (w/v) sildenafil citrate in 30% ethanol and 70% polyethylene glycol 400, and 1% (w/v) sildenafil citrate in 100% ethanol. (B) Transdermal diffusion rates of 1% (w/v) sildenafil citrate in 15% (w/v) isopropyl alcohol and water, 1% (w/v) sildenafil citrate in 23.5% (w/v) ethanol and water, 1% (w/v) sildenafil citrate in 20% (w/v) isopropyl alcohol and lactate buffer at pH 3.7, 1% (w/v) sildenafil citrate in 15% (w/v) isopropyl alcohol and lactate buffer at pH 3.7 and 1% sildenafil citrate in 23.5% (w/v) ethanol and lactate buffer pH 3.8.

### Examples

### Example 1

Mixtures of 5-25% (w/v) isopropyl alcohol and aqueous lactate buffer containing 0.1%, 2.1%, and 4.1% (w/v) lactic acid 90% were prepared by mixing the lactate buffer components with water and subsequently with isopropyl alcohol. Sildenafil citrate was dissolved in the mixture and the solubility of sildenafil citrate was determined in the mixture using a shake-flask method. Briefly, an excess amount of sildenafil citrate was stirred in the mixture overnight, the supernatant was separated from the solid material and the concentration of dissolved sildenafil citrate in the supernatant was analyzed by HPLC.

| **Formulation** | **001** | **002** | **003** | **004** | **005** |
|---|---|---|---|---|---|
| Isopropyl alcohol [% (w/v)] | 5 | 5 | 5 | 15 | 15 |
| Lactic acid 90% [% (w/v)] | 0.1 | 2.1 | 4.1 | 0.1 | 2.1 |
| pH value | 4.48 | 3.46 | 3.11 | 4.70 | 3.67 |
| Solubility of sildenafil citrate [% (w/w)] | 0.793 | 0.729 | 0.937 | 1.239 | 1.081 |

| **Formulation** | **006** | **007** | **008** | **009** |
|---|---|---|---|---|
| Isopropyl alcohol [% (w/v)] | 15 | 25 | 25 | 25 |
| Lactic acid 90% [% (w/v)] | 4.1 | 0.1 | 2.1 | 4.1 |
| pH value | 3.35 | 4.77 | 3.94 | 3.63 |
| Solubility of sildenafil citrate [% (w/w)] | 1.359 | 2.235 | 1.967 | 2.292 |

The solubility of sildenafil citrate is shown in Fig. 1A. As can be seen from the plot, a nearly consistent solubility of sildenafil citrate over a pH range of 3.1 - 4.8 is obtained.

Mixtures of 5 - 25% (w/v) ethanol 96% and aqueous lactate buffer containing 0.1%, 2.1%, and 4.1% (w/v) lactic acid 90% were prepared by mixing the lactate buffer components with water and subsequently with ethanol 96%. Sildenafil citrate was dissolved in the mixture and the solubility of sildenafil citrate was determined in the mixture.

| **Formulation** | **001** | **002** | **003** | **004** | **005** |
|---|---|---|---|---|---|
| Ethanol 96% [% (w/v)] | 5 | 5 | 5 | 15 | 15 |
| Lactic acid 90% [% (w/v)] | 0.1 | 2.1 | 4.1 | 0.1 | 2.1 |
| pH value | 4.56 | 3.46 | 3.12 | 4.73 | 3.64 |
| Solubility of sildenafil citrate [% (w/w)] | 0.815 | 0.681 | 0.900 | 0.990 | 0.817 |

| **Formulation** | **006** | **007** | **008** | **009** |
|---|---|---|---|---|
| Ethanol 96% [% (w/v)] | 15 | 25 | 25 | 25 |
| Lactic acid 90% [% (w/v)] | 4.1 | 0.1 | 2.1 | 4.1 |
| pH value | 3.32 | 4.88 | 3.9 | 3.57 |
| Solubility of sildenafil citrate [% (w/w)] | 1.073 | 1.415 | 1.214 | 1.569 |

The solubility of sildenafil citrate is shown in Fig. 1B. As can be seen from the plot, an acceptable solubility of sildenafil citrate over a pH range of 4-5 is obtained. Notably, the solubility does not decrease in the pH range of 4-5 and particularly towards pH 5, in contrast to solubility profiles of sildenafil citrate in water reported in the literature.

### Example 2

A pharmaceutical composition containing 1% (w/v) sildenafil citrate, 1.86% (w/v) sodium lactate 50%, 1.9% (w/v) lactic acid 90%, 15% (w/v) isopropyl alcohol, and ad 100% (w/v) purified water was prepared by mixing sodium lactate 50% and lactic acid 90% in purified water with isopropyl alcohol and dissolving sildenafil citrate in the mixture.

The pharmaceutical composition was subjected to stability analysis stored in glass vials at 25°C and 40°C for 24 weeks. The macroscopic appearance, microscopic appearance, pH value, assay of sildenafil citrate, and the impurities of sildenafil citrate was analyzed at the start, after 4 weeks, 8 weeks, 12 weeks, and 24 weeks. Macroscopic appearance was assessed by visual inspection, and microscopic appearance was assessed by inspection under a polarized light microscope (Zeiss Axio Imager). Assays for sildenafil citrate (i.e., sildenafil citrate concentration) and impurities were performed by HPLC analysis of the composition. The results are shown in the Table below.

| Parameter | Temp. | Start | 4 weeks | 8 weeks | 12 weeks | 24 weeks |
|---|---|---|---|---|---|---|
| Macroscopic appearance | 25°C | transparent | transparent | transparent | transparent | transparent |
| | 40°C | | transparent | transparent | transparent | transparent |
| Microscopic appearance | 25°C | no crystals | no crystals | no crystals | no crystals | no crystals |
| | 40°C | | no crystals | no crystals | no crystals | no crystals |
| pH value | 25°C | 3.70 | 3.71 | 3.71 | 3.68 | 3.71 |
| | 40°C | | 3.72 | 3.72 | 3.70 | 3.74 |
| Assay sildenafil citrate [g/100 g] | 25°C | 1.0422 | 1.0205 | 1.0250 | 1.0310 | 1.0296 |
| | 40°C | | 1.0110 | 1.0120 | 1.0206 | 0.9955 |
| Impurities sildenafil citrate [g/100 g] | 25°C | n. d. | 0.0009 | 0.0018 | 0.0036 | 0.008 |
| | 40°C | | 0.0036 | 0.0080 | 0.0149 | 0.0299 |

The parameters macroscopic appearance, microscopic appearance, pH value, assay sildenafil citrate, and impurities sildenafil citrate remained stable for the storage of at least 24 weeks.

The pharmaceutical composition containing 1% (w/v) sildenafil citrate, 1.86% (w/v) sodium lactate 50%, 1.9% (w/v) lactic acid 90%, 15% (w/v) isopropyl alcohol, and ad 100% (w/v) purified water was also subjected to photostress conditions. Photostress conditions were 1.2 MLux h, UV 200 W h/m², and a wavelength of 352 nm over six days. Results were compared to results for a solution of 0.08% (w/v) sildenafil citrate in purified water subjected to the same conditions.

The stability of both formulations under photostress conditions was assessed by HPLC analysis, and the results are shown in Figure 2.

The liquid topical pharmaceutical composition is stable under the photostress conditions whereas the solution of sildenafil citrate in purified water shows a significant decrease of the assay after six days.

### Example 3

Strat-M^{®} membranes within Franz diffusion cells were employed to assess the transdermal diffusion rate of sildenafil citrate from the investigated formulations as an indicator for the permeation potential.

Strat-M^{®} membranes are used for evaluation of the transdermal diffusion rate of APIs (Haq et al. Int J Pharm (2018) 547(1-2) 432-437).

The formulations were applied under infinite dosing in closed acceptor compartments of the Franz diffusion cells and the amount of sildenafil citrate diffused across Strat-M^{®} was determined within the acceptor phase composed of lactate buffer at pH 3.7 after 1, 2, 4, 6, and 24 hours.

The following compositions were compared, wherein all composition comprised 1% (w/v) sildenafil citrate:
- pharmaceutical composition containing 1% (w/v) sildenafil citrate, 1.86% (w/v) sodium lactate 50%, 1.9% (w/v) lactic acid 90%, 15% (w/v) isopropyl alcohol, and ad 100% (w/v) purified water (pH 3.7, Fig. 3A+B),
- pharmaceutical composition containing 1% (w/v) sildenafil citrate, 1.86% (w/v) sodium lactate 50%, 1.9% (w/v) lactic acid 90%, 23.5% (w/v) ethanol 96%, and ad 100% (w/v) purified water (pH 3.8, Fig. 3A+B),
- pharmaceutical composition containing 1% (w/v) sildenafil citrate, 1.86% (w/v) sodium lactate 50%, 1.9% (w/v) lactic acid 90%, 20% (w/v) isopropyl alcohol, and ad 100% (w/v) purified water (pH 3.7, Fig. 3B),
- comparative formulation composed of 1% (w/v) sildenafil citrate, 70% (v/v) polyethylene glycol and 30% (v/v) ethanol (Fig. 3A),
- comparative formulation of 1% (w/v) sildenafil citrate and 100% (v/v) ethanol (Fig. 3A)
- comparative formulation composed of 1% (w/v) sildenafil citrate, 15% (w/v) isopropyl alcohol, and ad 100% (w/v) purified water (Fig. 3B),
- comparative formulation composed of 1% (w/v) sildenafil citrate, 23.5% (w/v) ethanol, and ad 100% (w/v) purified water (Fig. 3B).

The transdermal diffusion rates are shown in Fig. 3A und Fig. 3B.

The pharmaceutical compositions according to the present invention exhibit a significantly lower transdermal diffusion rate than the formulations in 100% ethanol or 70% polyethylene glycole and 30% ethanol (Fig. 3A). They also exhibit a lower transdermal diffusion rate than the formulations using isopropyl alcohol or ethanol and purified water as solvent (Fig. 3B).

The present invention covers the following items:
1. A pharmaceutical composition comprising
   (A) 0.001-2.5% (w/w) of an active agent selected from sildenafil or a pharmaceutically acceptable salt thereof,
   (B) 5-25% (w/v) of at least one alcohol, and
   (C) at least 75% (w/v) of an aqueous buffer.
2. The pharmaceutical composition of item 1, wherein the active agent (A) is sildenafil citrate.
3. The pharmaceutical composition of any one of the preceding items, wherein the active agent (A) is stably dissolved in the pharmaceutical composition.
4. The pharmaceutical composition of any one of the preceding items, wherein the concentration of active agent (A) is 0.01-2.5% (w/w), preferably 0.1-2.5% (w/w), more preferably 0.5-2.5% (w/w), even more preferably 0.5-2.25% (w/w), particularly 0.75-1.25% (w/w).
5. The pharmaceutical composition of any one of the preceding items, wherein the at least one alcohol (B) is selected from C₂-C₄-alcohols having 1-3 hydroxy groups, or mixtures thereof, preferably selected from C₂-C₄-alcohols having 1-2 hydroxy groups, or mixtures thereof, more preferably selected from C₂-C₃-alcohols having 1 hydroxy group,
   wherein the alcohol is preferably saturated.
6. The pharmaceutical composition of any one of the preceding items, wherein the at least one alcohol (B) is selected from the group consisting of ethanol, isopropyl alcohol, 1-propanol, glycerol, propylene glycol, and 1,3-propanediol, or a mixture thereof, more preferably wherein the at least one alcohol (B) is isopropyl alcohol, ethanol, or a mixture thereof, in particular isopropyl alcohol.
7. The pharmaceutical composition of any one of the preceding items, wherein the concentration of the at least one alcohol (B) is 10-25% (w/v), preferably 10-20% (w/v).
8. The pharmaceutical composition of any one of the preceding items, wherein the weight ratio of active agent (A) to the at least one alcohol (B) is 1:4-1:50, preferably 1:8-1:25.
9. The pharmaceutical composition of any one of the preceding items, wherein the aqueous buffer (C) has a pH of 3.0-6.0, preferably 3.1-4.9, more preferably 3.3-4.5.
10. The pharmaceutical composition of any one of the preceding items, wherein the aqueous buffer (C) comprises as buffering agents at least one weak acid and one weak base, preferably wherein the aqueous buffer (C) comprises as buffering agents at least one weak acid and its conjugate base.
11. The pharmaceutical composition of item 10, wherein the buffering agents are organic compounds, preferably monoprotic or polyprotic organic weak acids and/or monoprotic or polyprotic organic weak bases, more preferably carboxylic acids and/or carboxylates or pharmaceutically acceptable salts thereof.
12. The pharmaceutical composition of item 10 or 11, wherein the at least one weak acid and optionally the conjugate acid of the at least one weak base have at least one pKₐ in the pH range of 3.0-6.0, more preferably 3.1-4.9, even more preferably 3.3-4.5.
13. The pharmaceutical composition of any one of items 10-12, wherein the at least one weak acid is selected from lactic acid, citric acid, succinic acid, acetic acid, tartaric acid or mixtures thereof and/or the at least one weak base is selected from pharmaceutically acceptable salts of lactate, citrate, succinate, acetate, tartrate or mixtures thereof, preferably wherein the weak base is the conjugate base of the at least one weak acid or a pharmaceutically acceptable salt thereof.
14. The pharmaceutical composition of any one of items 10-13, wherein the total concentration of buffering agents in the aqueous buffer (C) is 0.001-2 M, preferably 0.01-1.0 M, particularly 0.1-0.8 M.
15. The pharmaceutical composition of any one of the preceding items, wherein the aqueous buffer (C) is a lactate buffer, preferably comprising lactic acid and a lactate salt, wherein the lactate salt is preferably sodium lactate.
16. The pharmaceutical composition of item 15, wherein the lactate buffer has a total lactate concentration of 0.001-2 M, preferably 0.01-1.0 M, particularly 0.1-0.8 M.
17. The pharmaceutical composition of any one of items 15-16, wherein the lactate buffer comprises 0.1 - 4.1% (w/v) lactic acid and 0.05 - 3.1% (w/v) sodium lactate, preferably 0.5 - 3.0% (w/v) lactic acid and 0.2 - 2.5% (w/v) sodium lactate, in particular 1.0 - 2.5% (w/v) lactic acid and 0.5 - 2.0% (w/v) sodium lactate.
18. The pharmaceutical composition of any one of the preceding items, which is liquid, preferably which has a viscosity of 0.01-2000 mPa·s at 20°C.
19. The pharmaceutical composition of any one of the preceding items, which is provided as a solution, lotion, emulsion, suspension, foam, or shampoo.
20. The pharmaceutical composition of any one of the preceding items, which is for topical application, preferably for topical application to the skin.
21. The pharmaceutical composition of any one of the preceding items, which has a transdermal diffusion rate of active agent (A) of less than 300 pg/cm² in 24 hours across a Strat-M membrane under infinite dosing.
22. The pharmaceutical composition of any one of the preceding items, further comprising at least one additive selected from the group consisting of refatteners, preservatives, stabilizers, antioxidants, rheology modifiers, thickeners, coloring agents and any combination thereof.
23. The pharmaceutical composition of any one of the preceding items, further comprising at least one additional active agent selected from the group comprising menthol, biotin, zinc PCA, caffeine, niacinamide, panthenol, ectoine, ubiquinone-10, taurine, echinacea, tocopheryl acetate and combinations thereof.
24. A pharmaceutical composition according to any one of items 1-23 for use in a method of preventing and/or treating hair loss and/or female sexual dysfunction and/or erectile dysfunction.
25. The pharmaceutical composition for use according to item 24, which is for use in a method of preventing and/or treating hair loss,
   preferably wherein the hair loss is selected from androgenetic alopecia, telogen effluvium, alopecia areata, frontal fibrosing alopecia, and lichen planopilaris.
26. The pharmaceutical composition for use according to item 24, which is for use in a method of preventing and/or treating female sexual dysfunction,
   preferably wherein the female sexual dysfunction comprises female sexual arousal disorder, preferably selected from genital arousal disorder, subjective arousal disorder, and/or combined arousal disorder.

## Claims

1. A pharmaceutical composition comprising
(A) 0.001-2.5% (w/w) of an active agent selected from sildenafil or a pharmaceutically acceptable salt thereof,
(B) 5-25% (w/v) of at least one alcohol, and
(C) at least 75% (w/v) of an aqueous buffer.

2. The pharmaceutical composition of claim 1, wherein the active agent (A) is sildenafil citrate.

3. The pharmaceutical composition of any one of the preceding claims, wherein the active agent (A) is stably dissolved in the pharmaceutical composition, and/or wherein the concentration of active agent (A) is 0.01-2.5% (w/w), preferably 0.1-2.5% (w/w), more preferably 0.5-2.5% (w/w), even more preferably 0.5-2.25% (w/w), particularly 0.75-1.25% (w/w).

4. The pharmaceutical composition of any one of the preceding claims, wherein the at least one alcohol (B) is selected from C₂-C₄-alcohols having 1-3 hydroxy groups, or mixtures thereof, preferably selected from C₂-C₄-alcohols having 1-2 hydroxy groups, or mixtures thereof, more preferably selected from C₂-C₃-alcohols having 1 hydroxy group,
wherein the alcohol is preferably saturated,
more preferably wherein the at least one alcohol (B) is selected from the group consisting of ethanol, isopropyl alcohol, 1-propanol, glycerol, propylene glycol, and 1,3-propanediol, or a mixture thereof,
even more preferably wherein the at least one alcohol (B) is isopropyl alcohol, ethanol, or a mixture thereof, in particular isopropyl alcohol.

5. The pharmaceutical composition of any one of the preceding claims, wherein the concentration of the at least one alcohol (B) is 10-25% (w/v), preferably 10-20% (w/v), and/or
wherein the weight ratio of active agent (A) to the at least one alcohol (B) is 1:4-1:50, preferably 1:8-1:25.

6. The pharmaceutical composition of any one of the preceding claims, wherein the aqueous buffer (C) has a pH of 3.0-6.0, preferably 3.1-4.9, more preferably 3.3-4.5.

7. The pharmaceutical composition of any one of the preceding claims, wherein the aqueous buffer (C) comprises as buffering agents at least one weak acid and one weak base, preferably wherein the aqueous buffer (C) comprises as buffering agents at least one weak acid and its conjugate base,
particularly
wherein the buffering agents are organic compounds, preferably monoprotic or polyprotic organic weak acids and/or monoprotic or polyprotic organic weak bases, more preferably carboxylic acids and/or carboxylates or pharmaceutically acceptable salts thereof.

8. The pharmaceutical composition of claim 7, wherein the at least one weak acid and optionally the conjugate acid of the at least one weak base have at least one pKₐ in the pH range of 3.0-6.0, more preferably 3.1-4.9, even more preferably 3.3-4.5,
and/or
wherein the at least one weak acid is selected from lactic acid, citric acid, succinic acid, acetic acid, tartaric acid, or mixtures thereof and/or the at least one weak base is selected from pharmaceutically acceptable salts of lactate, citrate, succinate, acetate, tartrate, or mixtures thereof, preferably wherein the weak base is the conjugate base of the at least one weak acid or a pharmaceutically acceptable salt thereof,
and/or
wherein the total concentration of buffering agents in the aqueous buffer (C) is 0.001-2 M, preferably 0.01-1.0 M, particularly 0.1-0.8 M.

9. The pharmaceutical composition of any one of the preceding claims, wherein the aqueous buffer (C) is a lactate buffer, preferably comprising lactic acid and a lactate salt, wherein the lactate salt is preferably sodium lactate.

10. The pharmaceutical composition of claim 9, wherein the lactate buffer has a total lactate concentration of 0.001-2 M, preferably 0.01-1.0 M, particularly 0.1-0.8 M,
and/or
wherein the lactate buffer comprises 0.1 - 4.1% (w/v) lactic acid and 0.05 - 3.1% (w/v) sodium lactate, preferably 0.5 - 3.0% (w/v) lactic acid and 0.2 - 2.5% (w/v) sodium lactate, in particular 1.0 - 2.5% (w/v) lactic acid and 0.5 - 2.0% (w/v) sodium lactate.

11. The pharmaceutical composition of any one of the preceding claims, which is liquid, preferably which has a viscosity of 0.01-2000 mPa·s at 20°C,
and/or
which is provided as a solution, lotion, emulsion, suspension, foam, or shampoo.

12. The pharmaceutical composition of any one of the preceding claims, which is for topical application, preferably for topical application to the skin.

13. The pharmaceutical composition of any one of the preceding claims, which has a transdermal diffusion rate of active agent (A) of less than 300 pg/cm² in 24 hours across a Strat-M membrane under infinite dosing.

14. The pharmaceutical composition of any one of the preceding claims, further comprising at least one additive selected from the group consisting of refatteners, preservatives, stabilizers, antioxidants, rheology modifiers, thickeners, coloring agents and any combination thereof.

15. A pharmaceutical composition according to any one of claims 1-14 for use in a method of preventing and/or treating hair loss and/or female sexual dysfunction and/or erectile dysfunction,
wherein the hair loss is preferably selected from androgenetic alopecia, telogen effluvium, alopecia areata, frontal fibrosing alopecia, and lichen planopilaris, and/or wherein the female sexual dysfunction preferably comprises female sexual arousal disorder, more preferably selected from genital arousal disorder, subjective arousal disorder, and/or combined arousal disorder.
